# EUROPEAN PATENT APPLICATION

(11) **EP 4 502 612 A1**
(43) Date of publication of application: **05.02.2025**
(21) Application number: 23779689.1
(22) Date of filing: 16.03.2023
(51) Int. Cl.: G01N 35/00

(54) **INSPECTING DEVICE**

(30) Priority: 31.03.2022 JP 2022060858
(71) Applicant: FUJIFILM Corporation, Tokyo 106-8620 (JP)
(72) Inventor: TADA, Takuji, Ashigarakami-gun, Kanagawa 258-8538 (JP); HIBE, Daisuke, Ashigarakami-gun, Kanagawa 258-8538 (JP)
(74) Representative: Dehns Germany Partnerschaft mbB
(86) International application number: PCT/JP2023/010398
(87) International publication number: WO 2023/189671

(57) **Abstract**

The examination apparatus includes a dispensing unit that includes a nozzle through which a first liquid is discharged; a liquid tank that stores the first liquid or a second liquid; a heater that heats the first liquid or the second liquid; and a processor that controls the dispensing unit and the heater. The processor is configured to maintain a temperature of the heater at or above a predetermined first temperature during a period from end of the examination of one specimen to start of the examination of a next specimen, and measure a waiting time from the end of the examination of the one specimen to the examination of the next specimen, and execute, in accordance with the waiting time, a temperature control which is for compensating for a decrease in temperature caused by the waiting time, the temperature control including at least one of an empty discharge process of empty-discharging the first liquid or the second liquid from the dispensing unit to a place other than the reaction cell or a temperature setting change process of setting the heater to a second temperature higher than the first temperature.

## Description

### BACKGROUND OF THE INVENTION

### 1. Field of the Invention

The present disclosure relates to an examination apparatus.

### 2. Description of the Related Art

An examination apparatus (which is also referred to as an analysis apparatus) that quantitatively or qualitatively detects an examination target substance in a specimen is known. Many of such examination apparatuses use an immunoassay principle, and examples thereof include a chemiluminescent enzyme immunological analysis apparatus and a fluorescence immunological measurement apparatus.

Such an examination apparatus performs a detecting process of detecting an examination target substance in a specimen by detecting luminescence or fluorescence based on a label such as an enzyme label or a fluorescent label attached to the examination target substance in the specimen by using an immunoreaction. Further, before such a detecting process of the examination target substance, a pre-process such as labeling the examination target substance in the specimen is performed on the specimen. In some cases, the examination apparatus is configured to automatically execute the pre-process and the detecting process and output the detection result in a case where the pre-process and the detecting process are automated and a specimen collection container accommodating the collected specimen is loaded.

In order to attach a label to the examination target substance in the specimen, for example, such an automated examination apparatus performs the following pre-process using magnetic particles. First, in the reaction cell, the magnetic particles modified with the first binding substance (for example, the primary antibody) to specifically bind to the examination target substance (for example, the antigen) and the specimen are mixed with each other. Therefore, the examination target substance and the first binding substance are bound to each other to generate an immune complex. Thereby, the examination target substance is captured by the magnetic particles through the first binding substance. Thereafter, the immune complex (reacted component) is separated from the immune complex and a component derived from the specimen (unreacted component) that does not form the immune complex, that is, so-called bound free (B/F) separation is performed. In a case of the B/F separation, the mixed liquid is suctioned in a state where the magnetic particles are temporarily adsorbed to an inner wall surface of the reaction cell by a magnet disposed outside the reaction cell. Thereafter, a cleaning process is performed in which a cleaning liquid is discharged into the reaction cell, and the mixed liquid is suctioned and discharged in a state where the cleaning liquid and the magnetic particles are mixed. Next, a labeling reagent including a second binding substance (for example, a secondary antibody) to specifically bind to the examination target substance and the second binding substance bound to the label are mixed with the magnetic particles. Thereby, the examination target substance and the second binding substance captured on the magnetic particles through the first binding substance are bound to each other. As a result, the sandwich type immune complex, in which the examination target substance is interposed between the first binding substance and the second binding substance, is generated. Then, the cleaning process for B/F separation is performed again. In a case where the label is an enzyme label, the magnetic particles and the reagent including a luminescent substrate are further mixed and used for the examination process.

In such an examination apparatus, for example, a specific biological component or a chemical substance contained in a biological sample such as blood, serum, or urine is detected. In such a manner, in a case where a biological sample is used as a specimen, it is necessary to keep the temperature of the reagent used for pre-process or analysis of the specimen constant.

JP2020-106424A discloses an automatic analysis apparatus comprising a liquid temperature adjustment device that adjusts a temperature of a reagent supplied to a reaction container and that executes temperature adjustment of the temperature of the reagent on the basis of the detected temperature in the examination apparatus or the temperature of the reagent.

WO2019/087482A discloses an automatic analysis apparatus comprising a B/F separation unit that performs B/F separation, a detecting unit that detects a reacted component in a liquid after the B/F separation, and a temperature maintenance unit that maintains the B/F separation unit and the detecting unit in substantially the same temperature environment. In WO2019/087482A, the temperature maintenance unit performs temperature control on the basis of the temperature detected by the temperature sensor provided in the apparatus.

JP2017-37073A discloses a dispensing device provided with a heatable dispensing needle (nozzle) used for an analysis apparatus. The dispensing device disclosed in JP2017-37073A comprises two temperature sensors that are positioned at different distances from a nozzle distal end. The dispensing device is capable of dispensing a reagent of which a temperature has been appropriately controlled by discarding the reagent held by a dispensing nozzle in a case where the temperature detected by the two temperature sensors does not satisfy a predetermined condition.

WO2018/047545A proposes an analysis apparatus capable of suppressing a fluctuation in temperature of a reagent in a case where a nozzle is passed through, between a first specimen and a second and subsequent specimens after the analysis apparatus is switched from a waiting state to an analysis state, and making the temperature constant regardless of an analysis order. In the analysis apparatus disclosed in WO2018/047545A, in a case where the analysis is not continuous on the basis of information relating to whether or not the requested analysis is continuous, the temperature of the liquid is increased to supply the liquid to the nozzle. By supplying the heated liquid to the nozzle before suctioning the reagent, the nozzle is heated, and the reagent is suctioned after the nozzle is heated. Therefore, even in the first specimen after switching to the analysis state, the reagent can be dispensed at a stabilized temperature without lowering the temperature of the reagent passing through the nozzle.

### SUMMARY OF THE INVENTION

JP2020-106424A, WO2019/087482A, and JP2017-37073A disclose that temperature adjustment of the temperature of the reagent, temperature adjustment of the B/F separation unit and the detecting unit, or discarding of the reagent held by the dispensing nozzle is performed on the basis of the temperature detected by the temperature sensor. However, it is not considered that a difference in temperatures of the reagents in a case where the reagents pass through the nozzle occurs between the first specimen after the analysis apparatus is switched from the waiting state to the analysis state and the second and subsequent specimens. The difference in temperatures of the reagents to be dispensed may affect the examination results and may cause reduction in reliability of the examination results.

In WO2018/047545A, in a case where the analysis is not continuous on the basis of information relating to whether or not the requested analysis is continuous, the temperature of the liquid is increased to supply the liquid to the nozzle. However, the decrease in temperature corresponding to the waiting time between the analyses in a case where the analyses are not continuous is not considered. Therefore, in a case where the waiting time is long, there is a possibility that the temperature rise immediately after the waiting time is insufficient or the empty discharge for unnecessary temperature rise is performed. In a case where the temperature rise is insufficient, the reliability of the examination results may be reduced.

An object of the present disclosure is to provide an examination apparatus capable of obtaining an examination with higher reliability than that in the related art.

According to an aspect of the present disclosure, there is provided an examination apparatus that sequentially performs examination on a plurality of specimens, the examination apparatus comprising: a dispensing unit that includes a nozzle through which a first liquid is discharged into a reaction cell in which a specimen is accommodated; a liquid tank that stores the first liquid or a second liquid different from the first liquid; a heater that is disposed closer to a side of the liquid tank than the dispensing unit and heats the first liquid or the second liquid; and a processor that controls the dispensing unit and the heater, in which the processor is configured to maintain a temperature of the heater at or above a predetermined first temperature during a period from end of the examination of one specimen to start of the examination of a next specimen, and measure a waiting time from the end of the examination of the one specimen to the examination of the next specimen, and execute, in accordance with the waiting time, a temperature control which is for compensating for a decrease in temperature of the first liquid or a decrease in temperature of the nozzle caused by the waiting time, the temperature control including at least one of an empty discharge process of empty-discharging the first liquid or the second liquid from the dispensing unit to a place other than the reaction cell or a temperature setting change process of setting the heater to a second temperature higher than the first temperature.

In the examination apparatus according to the aspect of the present disclosure, the processor may be configured to set an empty discharge condition, which includes at least one of the number of discharge operations of the empty discharge or a single discharge amount of the empty discharge in the empty discharge process, in accordance with the waiting time.

In the examination apparatus according to the aspect of the present disclosure, the processor may be configured to, in the setting of the empty discharge condition, increase at least one of the number of discharge operations or the discharge amount as the waiting time increases.

In the examination apparatus according to the aspect of the present disclosure, the processor may be configured to set the number of discharge operations to 0 in a case where the waiting time is shorter than a preset threshold value in the setting of the empty discharge condition.

In the examination apparatus according to the aspect of the present disclosure, the processor may be configured to set the second temperature to be higher as the waiting time increases in the temperature setting change process.

In the examination apparatus according to the aspect of the present disclosure, the examination apparatus may further comprise: a temperature sensor that measures an ambient temperature, in which the processor is configured to execute the temperature control in accordance with the waiting time and the ambient temperature.

In the examination apparatus according to the aspect of the present disclosure, the processor may be configured to increase at least one of the number of discharge operations or the discharge amount as the waiting time increases and to increase at least one of the number of discharge operations or the discharge amount as the ambient temperature decreases in the setting of the empty discharge condition.

In the examination apparatus according to the aspect of the present disclosure, the processor may be configured to set the number of discharge operations to 0 in a case of satisfying a condition in which the waiting time and the ambient temperature are preset in the setting of the empty discharge condition.

In the examination apparatus according to the aspect of the present disclosure, the processor may be configured to set the second temperature to be higher as the waiting time increases and to set the second temperature to be higher as the ambient temperature decreases in the temperature setting change process.

In the examination apparatus according to the aspect of the present disclosure, the liquid tank may be a liquid tank that stores the first liquid, the heater may be a heater that heats the first liquid, and the processor may be configured to execute, in accordance with the waiting time, a temperature control which is for compensating for a decrease in temperature of the first liquid caused by the waiting time, the temperature control including at least one of the empty discharge process of empty-discharging the first liquid from the dispensing unit to the place other than the reaction cell or a setting change process of increasing a set temperature of the heater. In addition, here, the first liquid may be a cleaning liquid used for a cleaning process of separating an unreacted component from a reacted component in a reaction liquid obtained by reacting the specimen with a reagent.

According to the technology of the present disclosure, in the examination apparatus, it is possible to obtain an examination result having higher reliability than that in the related art.

### BRIEF DESCRIPTION OF THE DRAWINGS

Fig. 1 is a schematic diagram showing an overall configuration of an examination apparatus 10.
Fig. 2 is a diagram showing a procedure of chemiluminescent enzyme immunoassay.
Fig. 3 is a diagram showing a relationship between a waiting time t and a liquid temperature of a cleaning liquid 40 in a nozzle 14A.
Fig. 4 is a diagram showing a relationship between the number of discharge operations of the cleaning liquid 40 and the liquid temperature.
Fig. 5 is a diagram showing a relationship between the number of discharge operations of the cleaning liquid 40 and the liquid temperature in a case where a single discharge amount thereof is different.
Fig. 6 is a diagram showing a first examination flow.
Fig. 7 is a schematic diagram showing a correlation between the liquid temperature of the cleaning liquid and an amount of luminescence in a measurement device according to the related art.
Fig. 8 is a schematic diagram showing a change in liquid temperature of the cleaning liquid in a case where a continuous examination is performed in the measurement device according to the related art.
Fig. 9 is a schematic diagram showing a change in amount of luminescence in a case where a continuous examination is performed in the measurement device according to the related art.
Fig. 10 is a diagram showing a second examination flow.
Fig. 11 is a map showing the number of discharge operations which is set by the waiting time and an ambient temperature.
Fig. 12 is a diagram showing a relationship between the number of discharge operations and the liquid temperature immediately after a waiting state in a case where the temperature control is performed by the temperature setting change process and in a case where the temperature control is not performed.
Fig. 13 is a diagram showing a third examination flow.
Fig. 14 is a diagram showing a fourth examination flow.
Fig. 15 is a map showing a second temperature which is set by the waiting time and the ambient temperature.
Fig. 16 is a diagram showing a temperature setting change process procedure in the waiting state (idling state).
Fig. 17 is a diagram showing a flow from activation of the examination apparatus 10 to reception of a first examination.
Fig. 18 is a diagram showing an examination flow after an examination instruction is received in Fig. 17.
Fig. 19 is a diagram showing a flow in the waiting state after the end of the examination flow in Fig. 18.
Fig. 20 is a diagram for describing temperature control at the time of dispensing of a reagent.

### DESCRIPTION OF THE PREFERRED EMBODIMENTS

Hereinafter, an examination apparatus according to an embodiment of the present disclosure will be described with reference to the drawings. In each of the drawings, constituent elements represented by the same reference numerals mean the same constituent elements. However, unless otherwise specified in the specification, each constituent element is not limited to one, and each constituent element may be plural.

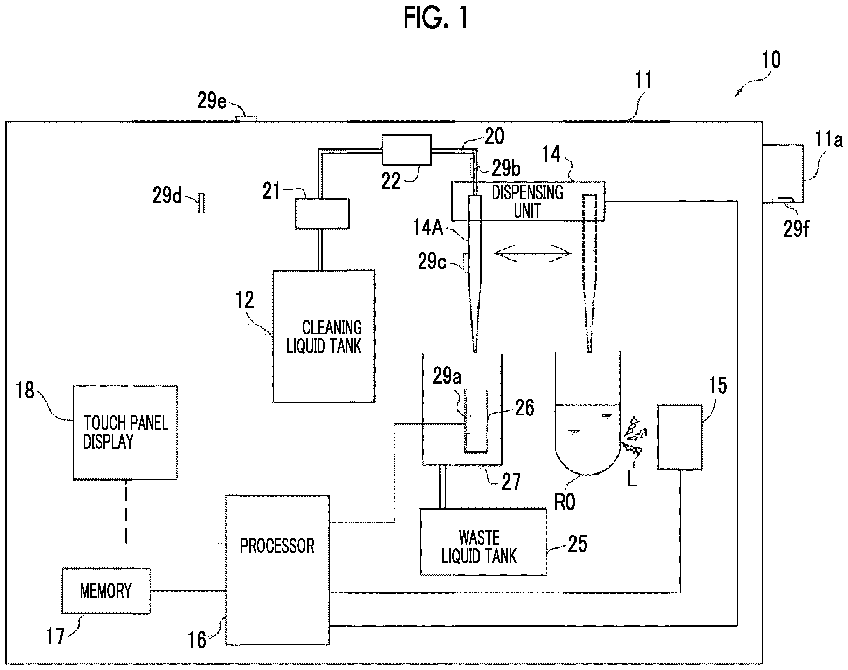

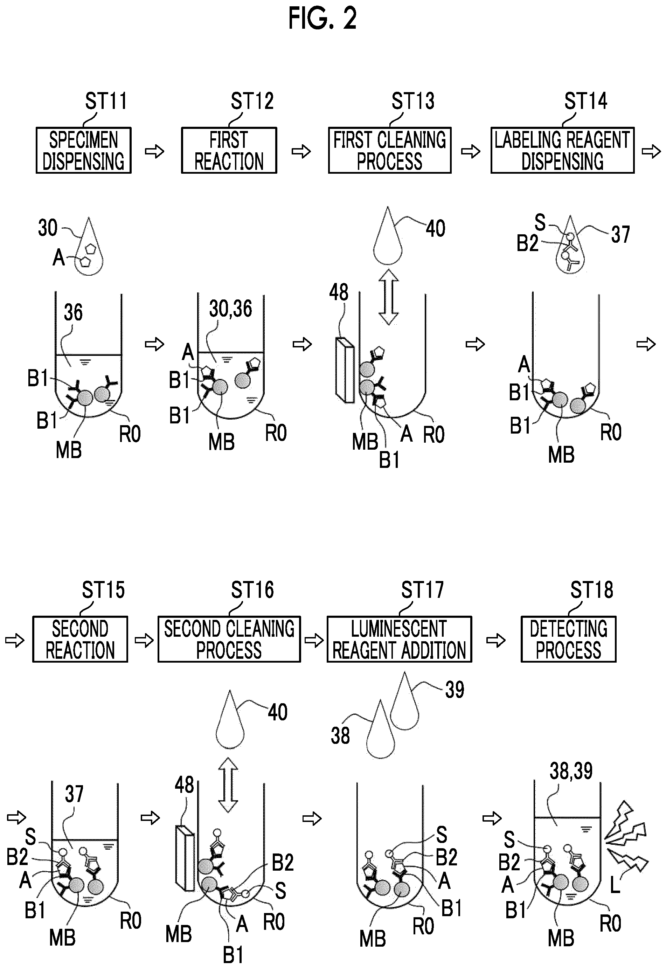

Fig. 1 is a schematic diagram showing an overall configuration of an examination apparatus 10 according to the embodiment of the present disclosure. For example, the examination apparatus 10 is an immunological analysis apparatus that detects an examination target substance A in a specimen 30 (refer to Fig. 2) collected from a biological body by using an antigen-antibody reaction. The examination apparatus 10 optically detects the examination target substance A in the specimen 30 using a reaction cell R0, and outputs an examination result. The examination apparatus 10 sequentially performs examinations on the specimen 30.

The specimen 30 is, for example, a body fluid such as blood collected from a biological body. In a case where the specimen 30 is blood, the specimen 30 may be any of whole blood, blood plasma, serum, and the like. Further, a centrifuge may be provided in the examination apparatus 10 to extract blood plasma or serum from whole blood. Furthermore, the examination target substance A that can be included in the specimen 30 is an antigen, an antibody, a protein, a low-molecular-weight compound, and the like. It should be noted that the specimen 30 is not limited to blood, and may be a substance collected from a biological body, such as urine or body fluid.

The examination apparatus 10 of the present example performs an examination based on a chemiluminescent enzyme immunoassay method. First, the procedure of the chemiluminescent enzyme immunoassay of the present example will be described with reference to Fig. 2. Fig. 2 schematically shows the reaction in the reaction cell R0 in a case where the specimen 30 includes the examination target substance A.

The reaction cell R0 accommodates the magnetic particles MB modified with a first binding substance B1 in advance, and a buffer solution 36 is dispensed into the reaction cell R0 before the dispensing of the specimen 30. In this state, the specimen 30 is dispensed into the reaction cell R0 (Step ST11).

In the reaction cell R0, the magnetic particles MB, the specimen 30, and the buffer solution 36 are mixed with each other, and as the first reaction, a binding reaction, in which the examination target substance A in the specimen 30 and the first binding substance B1 are specifically bound to each other, occurs (Step ST12). In the first reaction, the examination target substance A in the specimen 30 binds to the first binding substance B1. Therefore, the examination target substance A is captured by the magnetic particles MB through the first binding substance B1 interposed therebetween.

Next, a first cleaning process (B/F separation) for removing unreacted components other than the examination target substance A captured by the magnetic particles MB is performed (Step ST13). The magnet 48 are disposed close to the outside of the reaction cell R0, the reaction liquid after the first reaction is discharged in a state where the magnetic particles MB are collected on the inner wall surface of the reaction cell R0, and then a cleaning liquid 40 is dispensed into the reaction cell R0. In Step ST13 in Fig. 2, the bidirectional arrow in the up-down direction shown at the upper portion of the reaction cell R0 schematically indicates a state in which the cleaning liquid 40 is dispensed into the reaction cell R0 and the cleaning liquid 40 is discharged from the reaction cell R0. In the first cleaning process, the dispensing and the discharging of the cleaning liquid 40 are repeated a plurality of times. The cleaning liquid 40 is also discharged from the reaction cell R0 in a state where a magnet 48 is disposed close to the outside of the reaction cell R0 and the magnetic particles MB are collected on the inner wall surface of the reaction cell R0.

After the first cleaning process, a labeling reagent 37 is dispensed into the reaction cell R0 (Step ST14). The labeling reagent 37 includes a second binding substance B2 to which a label S is attached and which is a binding substance that specifically binds to the examination target substance A.

In the reaction cell R0, as the second reaction, a binding reaction, in which the examination target substance A captured by the magnetic particles MB and the second binding substance B2 specifically bind to each other, occurs (Step ST15). Thereby, the label S is attached to the examination target substance A through the second binding substance B2.

Next, the second cleaning process (B/F separation), which is for removing the unreacted components other than the second binding substance B2 bound to the examination target substance A captured by the magnetic particles MB in the labeling reagent 37, is performed (Step ST16). The second cleaning process (Step ST16) is performed in the same manner as the first cleaning process (Step ST13).

Thereafter, a first luminescent reagent 38 and a second luminescent reagent 39 are added to the reaction cell R0 (Step ST17). The label S is an enzyme and causes chemiluminescence L in the presence of the first luminescent reagent 38 and the second luminescent reagent 39 including a luminescent substrate. Then, the examination target substance A is detected by detecting the chemiluminescence L (Step ST18).

The above-mentioned chemiluminescent immunoassay method is performed in the examination apparatus 10. That is, in the examination apparatus 10, a pre-process of dispensing various reagents to the respective individual specimens 30 is performed (Steps ST11 to ST17), and a detecting process of the chemiluminescence L caused by the label S attached to the examination target substance A is performed (Step ST18).

As shown in Fig. 1, the examination apparatus 10 comprises, for example, a cleaning liquid tank 12, a dispensing unit 14, a photodetector 15, a processor 16, a memory 17, and a touch panel display 18.

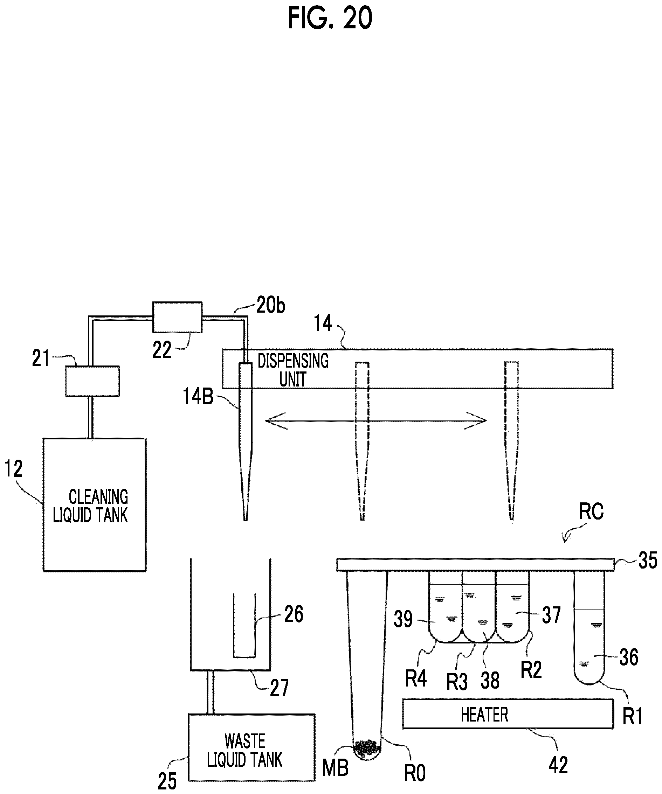

The dispensing unit 14 performs a process of dispensing and mixing of liquids such as the above-mentioned buffer solution 36, the labeling reagent 37, the first luminescent reagent 38, the second luminescent reagent 39, and the cleaning liquid 40. The buffer solution 36, the labeling reagent 37, the first luminescent reagent 38, and the second luminescent reagent 39 used for single examination are provided in a cartridge (here, refer to Fig. 20) not shown in Fig. 1. The dispensing unit 14 appropriately suctions each of the liquids 36 to 39 from the cartridge and dispenses the liquids into the reaction cell R0. On the other hand, the cleaning liquid 40 is stored in the cleaning liquid tank 12. The cleaning liquid 40 is an example of a first liquid, and the cleaning liquid tank 12 is an example of the liquid tank. In addition, in the following description, in a case where it is not necessary to distinguish the buffer solution 36, the labeling reagent 37, the first luminescent reagent 38, and the second luminescent reagent 39 from each other, these are collectively referred to as the reagents 36 to 39.

The dispensing unit 14 comprises a nozzle 14A that discharges the cleaning liquid 40 supplied from the cleaning liquid tank 12 into the reaction cell R0. The nozzle 14A is a nozzle for dispensing the cleaning liquid 40 into the reaction cell R0 in at least the second cleaning process. The dispensing unit 14 comprises a nozzle (not shown in the drawing) for suctioning and dispensing the reagents 37 to 39 or the like separately from the nozzle 14A, and a nozzle (not shown in the drawing) for dispensing the cleaning liquid 40 into the reaction cell R0 in the first cleaning process. A base end side of the nozzle 14A is connected to a pipe 20 connected to the cleaning liquid tank 12, and the cleaning liquid 40 is supplied to the nozzle 14A from the base end side. The dispensing unit 14 has a discharge mechanism (not shown in the drawing) such as a pump for discharging the cleaning liquid 40 from the nozzle 14A.

The examination apparatus 10 comprises a first heater 21 and a second heater 22 for increasing a temperature of the cleaning liquid 40 in the middle of the pipe 20 that connects the cleaning liquid tank 12 and the dispensing unit 14. The first heater 21 and the second heater 22 are disposed closer to the side of the cleaning liquid tank 12 than the dispensing unit 14, and are provided to heat the cleaning liquid 40 to adjust the temperature of the cleaning liquid 40 discharged into the reaction cell R0 through the dispensing unit 14 to a target temperature. Here, a configuration in which two heaters are provided is adopted, but only one heater may be provided, or three or more heaters may be provided.

The photodetector 15 is a photomultiplier tube, a photodiode, or the like. The photodetector 15 is disposed to face the reaction cell R0 and detects the chemiluminescence L caused by the label S bound to the examination target substance A. The photodetector 15 outputs a received light signal corresponding to an amount of received light to the processor 16.

A processor 16 integrally controls each unit of the examination apparatus 10. An example of the processor 16 is a central processing unit (CPU) that performs various types of control by executing a program. The CPU functions as a control unit that controls each unit by executing a program. Further, the processor 16 detects whether or not the specimen 30 includes the examination target substance A and the concentration thereof on the basis of the received light signal which is output by the photodetector 15. Further, as will be described later, the processor 16 executes temperature control of the cleaning liquid 40.

A memory 17 is an example of a memory connected to or built into the CPU as the processor 16. For example, the memory 17 stores various programs such as a program for controlling the examination apparatus 10 and a program for executing the detecting process. Further, the memory 17 stores setting information used by the processor 16 to perform various controls, in addition to a control program.

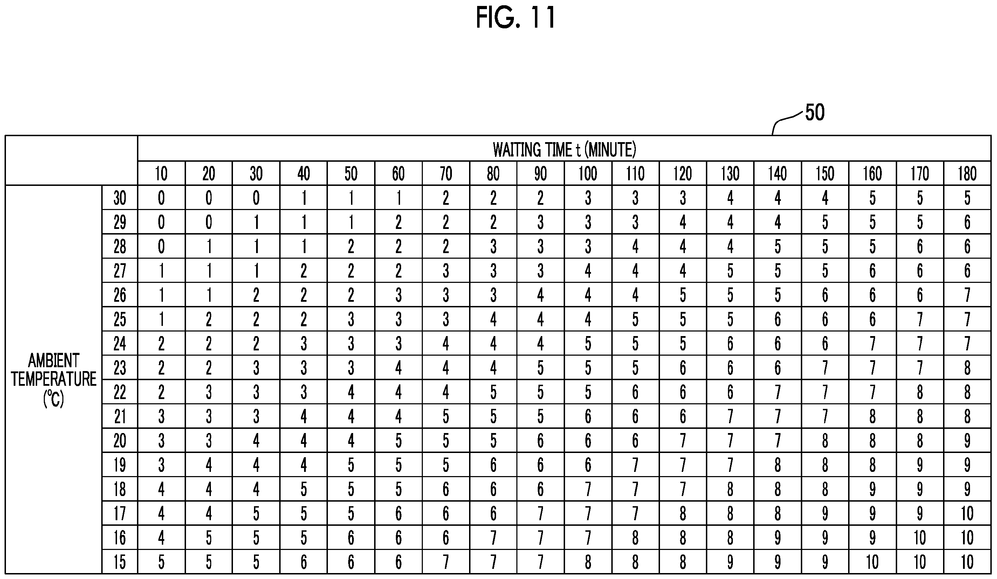

The setting information includes, for example, information indicating a correspondence relationship between the light amount of the chemiluminescence L and an amount of the examination target substance, and a map 50 (refer to Fig. 11) used for setting of the empty discharge condition for temperature control of the cleaning liquid 40 or setting of the heater temperature.

The touch panel display 18 receives an operation instruction, such as an instruction to start an examination, from a user. Further, the touch panel display 18 displays information such as an examination result. As described above, the touch panel display 18 functions as an operation instruction receiving unit that receives the operation instruction and the display unit that displays various types of information.

Since the specimen 30 is a biological sample, in the examination apparatus 10, the reagents 36 to 39 and the like to be mixed with the specimen 30 and the cleaning liquid 40 are subjected to temperature control so as to reach the target temperature, for example, 37°C when the reagents 36 to 39 and the like and the cleaning liquid 40 are dispensed into the reaction cell R0.

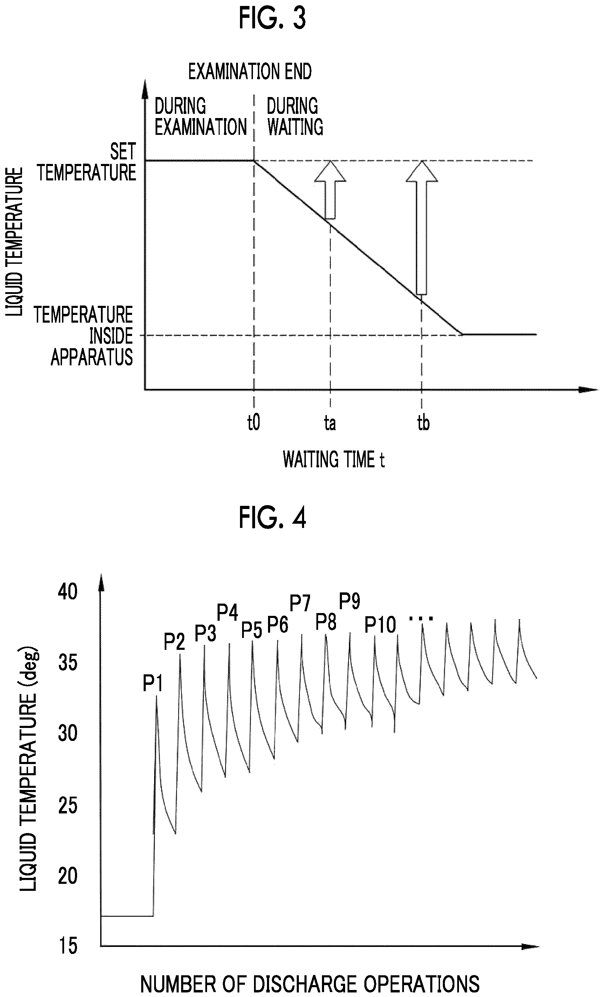

The cleaning liquid 40 stored in the cleaning liquid tank 12 is supplied to the nozzle 14A of the dispensing unit 14 through the pipe 20. The cleaning liquid 40 is supplied to the nozzle 14A after being heated by the first heater 21 and the second heater 22. While the examination apparatus 10 is being activated, the first heater 21 and the second heater 22 are basically maintained at a predetermined set temperature. In the examination apparatus 10, in a case where the examinations of the plurality of specimens 30 are continuously performed, the pipe 20 and the nozzle 14A are warmed by the heated cleaning liquid 40 passing therethrough to the same temperature as the temperature of the cleaning liquid 40. However, in a case where there is a waiting time between the examination of one specimen 30 and the examination of the next specimen 30, the temperature of the pipe 20, the nozzle 14A, and the cleaning liquid 40 held therein gradually decreases (refer to Fig. 3).

The processor 16 maintains the temperature of the second heater 22 at the predetermined first temperature T1 or higher until the examination of one specimen 30 ends and the examination of the next specimen 30 starts. Further, the processor 16 measures a waiting time t from the end of the examination on one specimen 30 to the start of the examination on the next specimen 30. In addition, the processor 16 executes the temperature control for compensating for the decrease in temperature of the cleaning liquid 40 in accordance with the waiting time t. In the present embodiment, the temperature control is an empty discharge process of performing empty discharge to a place other than the reaction cell R0, in which the temperature of the cleaning liquid 40 in the dispensing unit 14 is lowered. The processor 16 executes the empty discharge process in accordance with the waiting time t.

It should be noted that in the present example, the first heater 21 and the second heater 22 are maintained at a preset temperature. For example, in the first heater 21 and the second heater 22, the temperature of the first heater 21 on the cleaning liquid tank 12 side is set to be lower than the temperature of the second heater 22 on the dispensing unit 14 side.

The examination apparatus 10 comprises a waste liquid tank 25 for storing the cleaning liquid 40 empty-discharged from the nozzle 14A. The waste liquid tank 25 is connected to an accommodation tank 27 that accommodates a cleaning pod 26 for cleaning the nozzle 14A. The cleaning liquid 40 is empty-discharged to the cleaning pod 26, the cleaning liquid 40 overflowing from the cleaning pod 26 flows into the waste liquid tank 25 through the accommodation tank 27, and the cleaning liquid 40 is stored in the waste liquid tank 25.

The empty discharge process as the temperature setting change process performed by the processor 16 will be described in more detail.

Fig. 3 shows a relationship between the waiting time t and the liquid temperature of the cleaning liquid 40 in the nozzle 14A. During the examination, the temperature of the cleaning liquid 40 of the nozzle 14A is a substantially set temperature of the heater. From the time point t0 at which the examination ends, the liquid temperatures of the cleaning liquid 40 in the nozzle 14A and the pipe portion 20A start to decrease in order to dissipate heat to the surrounding environment. The liquid temperature gradually decreases with the passage of time, finally reaches a temperature of air in the apparatus, and becomes substantially constant. It should be noted that the same temperature change occurs in the nozzle 14A and the pipe portion 20A that hold the cleaning liquid 40. As shown in Fig. 3, naturally, a degree of decrease in liquid temperature is different between a time point ta at which the waiting time point t is relatively short and a time point tb at which the waiting time t is relatively long.

In the empty discharge process, the pipe portion 20A from the second heater 22 to the nozzle 14A and the nozzle 14A are warmed by discharging the cleaning liquid 40 of the target temperature by empty-discharging the cleaning liquid 40. The lower the temperatures of the nozzle 14A and the pipe portion 20A at the start of the next examination, the greater the temperatures need to be increased to the target temperatures. In a case where the temperature of the second heater 22 is constant, in order to further increase the temperature, it is necessary to increase the total discharge amount by increasing the number of empty discharge operations of the cleaning liquid 40 or by increasing the single discharge amount. As shown in Fig. 3, a case where the discharging is started at the time point tb at which the waiting time is relatively long and the time point ta at which the waiting time is relatively short will be considered. At the time point tb, the decreases in temperatures of the pipe portion 20A and the nozzle 14A is larger than that at the time point ta. Therefore, it is necessary to flow a larger amount of the cleaning liquid 40 than in a case where the discharging is started at the time point ta in order to reach the target temperature.

Fig. 4 shows a relationship between the number of discharge operations of the empty discharge of the cleaning liquid 40 and the liquid temperature at that time after the waiting state. The liquid temperature is measured by a temperature sensor 29a which is provided in the cleaning pod 26. Peak values indicated by P1, P2, ..., and Pn shown in Fig. 4 are liquid temperatures at the time of the first, second, ..., and n-th discharge operations. As shown in Fig. 4, the liquid temperature becomes closer to the target temperature (here, 37°C) each time the number of discharge operations is incremented. Then, after a certain number of discharge operations, the target temperature is stabilized at a substantially target temperature.

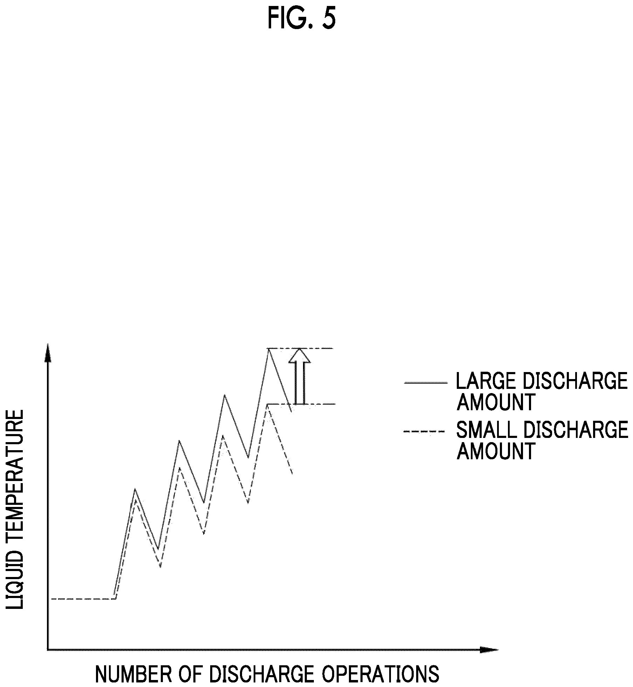

Fig. 5 shows a relationship between the number of discharge operations and the liquid temperature in a case where the discharge amount per one empty discharge operation was relatively large and in a case where the discharge amount per one empty discharge operation was relatively small. As shown in Fig. 5, in a case where the number of discharge operations is the same, the higher the discharge amount, the faster the discharged liquid temperature increases.

In such a manner, in a case where the processor 16 executes the empty discharge process of empty-discharging the cleaning liquid 40 of which the temperature is decreased in the dispensing unit 14 to a place other than the reaction cell R0 as the temperature control, it is preferable that the processor 16 sets the empty discharge condition including at least one of the number of discharge operations of the empty discharge or the single discharge amount of the empty discharge in the empty discharge process in accordance with the waiting time t. For example, in the setting of the empty discharge condition, the processor 16 increases at least one of the number of discharge operations or the discharge amount as the waiting time t is longer.

In the examination apparatus 10 having the above-mentioned configuration, as a first examination flow in a case where the examination is performed on the specimen 30, an examination flow in a case where the processor 16 executes the temperature control including the empty discharge process in accordance with the waiting time t will be described.

### (First Examination Flow)

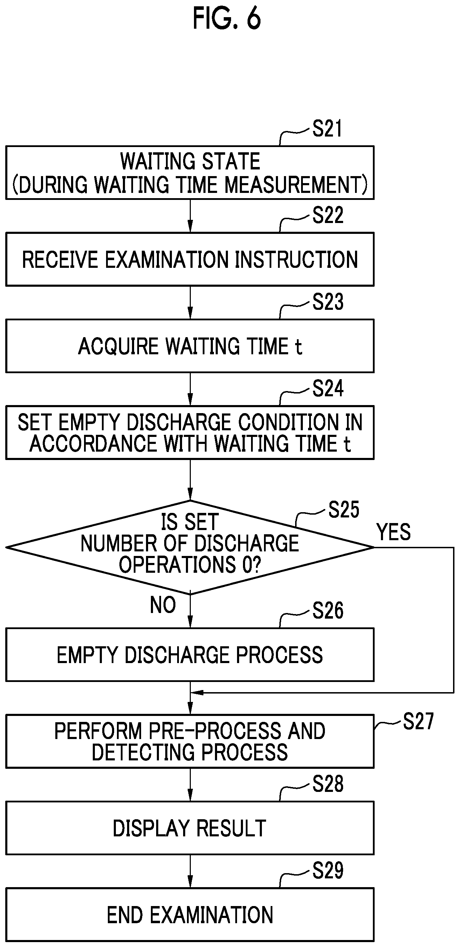

Fig. 6 is a diagram showing the first examination flow. The first examination flow is a process procedure in a case where the examination apparatus 10 is activated and an examination instruction is received in a waiting state (idle state) after the previous examination ends. It should be noted that, in a case where the examination apparatus 10 is activated, the first heater 21 is set to 35°C and the second heater 22 is set to 38°C (first temperature T1) as an example. In the present example, the temperatures of the first heater 21 and the second heater 22 are not changed and are kept constant.

In the waiting state, the processor 16 measures the waiting time from the end of the previous examination (Step S21). In a case where an examination instruction for the next examination is received (Step S22), the processor 16 acquires the waiting time t from the end of the previous examination (Step S23).

The processor 16 sets the empty discharge condition including at least one of the number of discharge operations of the empty discharge or the single discharge amount of the empty discharge in the empty discharge process in accordance with the waiting time t (Step S24). In the setting of the empty discharge condition (Step S24), the processor 16 determines whether or not the waiting time t is shorter than a preset threshold value α, and sets the number of discharge operations to 0 in a case where the waiting time t is shorter than the threshold value α. On the other hand, in a case where the waiting time t is equal to or longer than the threshold value α, the processor 16 performs a setting by which at least one of the number of discharge operations or the discharge amount is increased as the waiting time t is longer, in the setting of the empty discharge condition (Step S24).

The waiting time t and the map of either the number of discharge operations or the discharge amount may be stored in the memory 17, and the processor 16 may determine the empty discharge condition with reference to the map.

Thereafter, in a case where the waiting time t is less than the preset threshold value α, that is, in a case where the set number of discharge operations is 0 (Step S25: YES), the processor 16 performs the pre-process and a photodetecting process without performing the empty discharge process (Step S27). In a case where the set number of discharge operations is not 0 (Step S25: NO), the processor 16 controls the dispensing unit 14 such that the dispensing unit 14 performs the empty discharge process of empty-discharging the cleaning liquid 40 from the nozzle 14A in accordance with the set empty discharge condition (Step S26).

Then, the processor 16 controls the dispensing unit 14 such that the dispensing unit 14 performs the pre-process (Step ST11 to ST17 in Fig. 2), and controls the photodetector 15 such that the photodetector 15 performs the detecting process (Step ST18 in Fig. 2) (Step S27). It should be noted that, in the procedure of the chemiluminescent enzyme immunoassay described with reference to Fig. 2, the process of the second reaction (Step ST15 in Fig. 2) may be performed in parallel in a period from the reception of the examination instruction (Step S22) to the empty discharge process (Step S26). In particular, it is preferable that the empty discharge process (Step S26) is performed immediately before the second cleaning process (Step ST16 in Fig. 2), and the second cleaning process is performed immediately after the empty discharge process (Step S26).

The processor 16 detects a concentration of the examination target substance A on the basis of the received light signal from the photodetector 15, displays the examination result on the touch panel display 18 (Step S28), and ends the examination (Step S29). After the end of the examination (Step S29), in a case where the examination of the next specimen 30 is not performed, the examination apparatus 10 returns to the first waiting state (Step S21), and the processor 16 measures the waiting time.

As described above, the examination apparatus 10 according to the present embodiment comprises the dispensing unit 14 that has the nozzle 14A through which the cleaning liquid (an example of the first liquid) is discharged into the reaction cell R0, the cleaning liquid tank 12 (an example of the liquid tank) that stores the cleaning liquid 40 (an example of the first liquid), the first heater 21 and the second heater 22 that are disposed closer to the cleaning liquid tank 12 side than the dispensing unit 14 and that heat the cleaning liquid 40, and the processor 16 that controls the dispensing unit 14 and the heaters 21 and 22. The processor 16 maintains the temperature of the second heater 22 at the predetermined first temperature or higher until the examination of one specimen 30 ends and the examination of the next specimen 30 is started, and measures the waiting time t from the end of the examination of one specimen 30 to the start of the examination of the next specimen 30. Then, in a case of the examination of the next specimen 30, in the temperature control that compensates for the decrease in temperature of the cleaning liquid 40 occurring in accordance with the waiting time t, the empty discharge process of empty-discharging the cleaning liquid 40, of which the temperature has decreased in the dispensing unit 14 to a place other than the reaction cell R0 from the dispensing unit 14, is executed in accordance with the waiting time t. The empty discharge process is executed in accordance with the waiting time t. Therefore, it is possible to discharge the cleaning liquid 40 having an appropriate temperature at which a difference in temperature from the target temperature is suppressed as compared with the related art into the reaction cell R0 even in the examination immediately after the waiting time. Therefore, it is possible to obtain a highly reliable examination result even for the examination immediately after the waiting state.

Even in the examination apparatus according to the related art in which the temperature control is not performed, in a case where the examination of the next specimen is performed from the waiting state, the cold cleaning liquid 40 in the nozzle 14A and the pipe 20 is empty-discharged, and then the examination process is executed. However, in the examination apparatus according to the related art, the number of discharge operations and the discharge amount of the empty discharge are fixed by determining the empty discharge conditions such as the number of discharge operations and the single discharge amount of the empty discharge such that the examination apparatus can cope with a case where the waiting time is relatively long. Accordingly, the examination apparatus is not configured such that the empty discharge conditions are changed. Therefore, in a case where the waiting time t is short, excessive empty discharge may be performed, and the cleaning liquid 40 may be wasted. On the other hand, in the examination apparatus 10 according to the above-mentioned embodiment, the empty discharge process is executed in accordance with the waiting time t. Therefore, it is possible to suppress excessive empty discharge and to reduce the amount of the cleaning liquid 40 to be used.

In the above-mentioned embodiment, the processor 16 increases at least one of the number of discharge operations or the discharge amount as the waiting time t is longer in the setting of the empty discharge condition. As described above, as the waiting time t is longer, the temperature of the nozzle 14A or the pipe 20 and the cleaning liquid 40 held in the nozzle 14A or the pipe 20 is lower. Therefore, by increasing at least one of the number of discharge operations or the discharge amount and increasing the total discharge amount, it is possible to discharge the cleaning liquid 40, which has an appropriate temperature stabilized even in the examination immediately after the waiting state, into the reaction cell R0.

In the above-mentioned embodiment, in a case where the processor 16 sets the number of discharge operations to 0 in a case where the waiting time t is preset and is shorter than the threshold value in the setting of the empty discharge condition. Thereby, the amount of the cleaning liquid 40 used can be reduced.

In the above-mentioned embodiment, the temperature control of the cleaning liquid 40 used in the second cleaning process has been described as an example. However, the liquid, on which the temperature control according to the technology of the present disclosure is executed, is not limited to the cleaning liquid 40 used in the second cleaning process. The same temperature control may be performed on the cleaning liquid 40 used in the first cleaning process. Further, in a case where various reagents such as a buffer solution, a labeling reagent, and a luminescent reagent are also configured to be supplied to the nozzle through a pipe from a liquid tank for storing each of the reagents, the reagents may be discharged from the nozzle to the reaction cell R0 at the target temperature. Thus, the same temperature control may be performed.

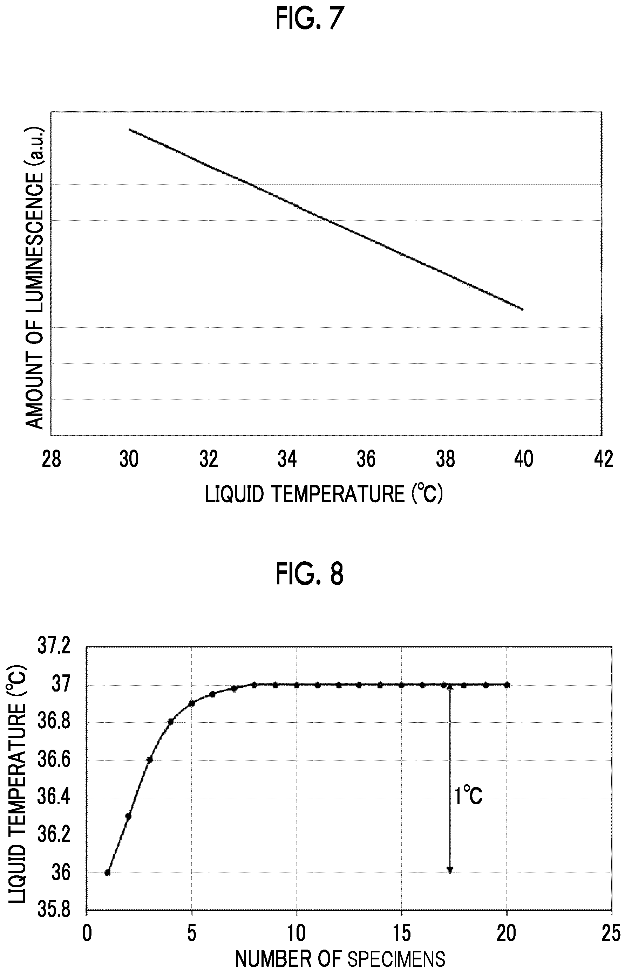

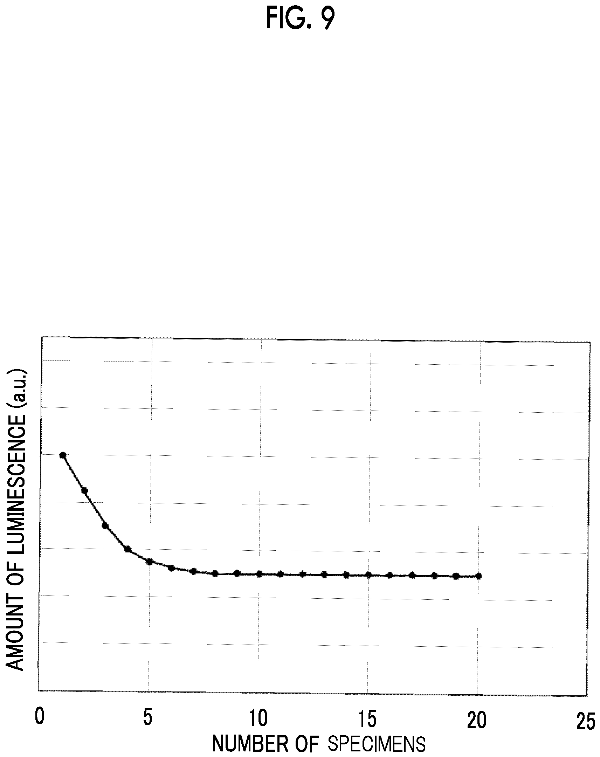

The present inventors found the following. In the chemiluminescent enzyme immunoassay using the examination apparatus according to the related art, with a certain reagent, examination is performed on a correlation between the liquid temperature of the second cleaning liquid and the amount of luminescence. Then, the correlation is negative, as shown in Fig. 7. As described above, in the second reaction, the specific binding between the examination target substance A and the second binding substance B2 occurs. Although a part of the second binding substance B2 is dissociated from the examination target substance A due to the cleaning operation, the higher the liquid temperature, the larger the amount of dissociation. Thus, it would appear that the negative correlation as shown in Fig. 7 occurs. In a case where the inclination of the graph in Fig. 7 is a/°C and the liquid temperature changes by 1°C during continuous measurement as shown in Fig. 8, the change in amount of luminescence as shown in Fig. 9 is observed. Even in a case where the same specimen is measured, the amount of luminescence is changed by a. As a result, there is a concern about the reliability of the examination.

According to the examination apparatus 10 of the present embodiment, the cleaning liquid 40 of which the temperature is controlled in accordance with the waiting state is dispensed into the reaction cell R0. Therefore, a decrease in liquid temperature can be suppressed. The examination can be performed at a stable liquid temperature. Therefore, the fluctuation in amount of luminescence due to the change in liquid temperature can be suppressed, and a highly reliable examination result can be obtained.

As a modification example, the examination apparatus 10 may comprise at least one of temperature sensors 29a to 29f that measure the ambient temperature, and the processor 16 may execute the temperature control in accordance with the waiting time t and the ambient temperature. The ambient temperature has an effect on the temperature of the nozzle 14A or the liquid held in the nozzle 14A.

The temperature sensor 29 is a thermocouple, a thermistor, or the like, and outputs the measured temperature to the processor 16. The ambient temperature may be a temperature that has an effect on the temperature of the cleaning liquid 40 to be discharged into the reaction cell R0. The ambient temperature is, for example, a temperature (liquid temperature) of the cleaning liquid 40 that is empty-discharged to the inside of the cleaning pod 26, a temperature of the pipe 20, a temperature of the nozzle 14A, an air temperature in the examination apparatus 10, an air temperature outside the examination apparatus 10, and the like. The temperature of the cleaning liquid 40 empty-discharged to the inside of the cleaning pod 26 can be measured by the temperature sensor 29a disposed in the cleaning pod 26. The temperature of the pipe 20 can be measured by the temperature sensor 29b provided in the pipe 20. The temperature of the nozzle 14A can be measured by the temperature sensor 29c provided in the nozzle 14A. The air temperature in the examination apparatus 10 can be measured by the temperature sensor 29d disposed in the housing 11 of the examination apparatus 10. In such a case, the temperature sensor 29d may be disposed at any position in the housing 11. The air temperature outside the examination apparatus 10 can be measured by the temperature sensor 29e attached to the outside of the housing 11 of the examination apparatus 10 or the temperature sensor 29f provided in an air inlet 11a. In practice, at least one of the temperature sensors 29a to 29f may be provided. It should be noted that, in a case where it is not necessary to distinguish the temperature sensors 29a to 29f from each other, the temperature sensors 29a to 29f will be simply referred to as the temperature sensor 29.

In the examination apparatus 10 having the above-mentioned configuration, as a second examination flow in a case where the examination is performed on the specimen 30, an examination flow in a case where the processor 16 executes the temperature control including the empty discharge process in accordance with the waiting time t and the ambient temperature will be described.

### (Second Examination Flow)

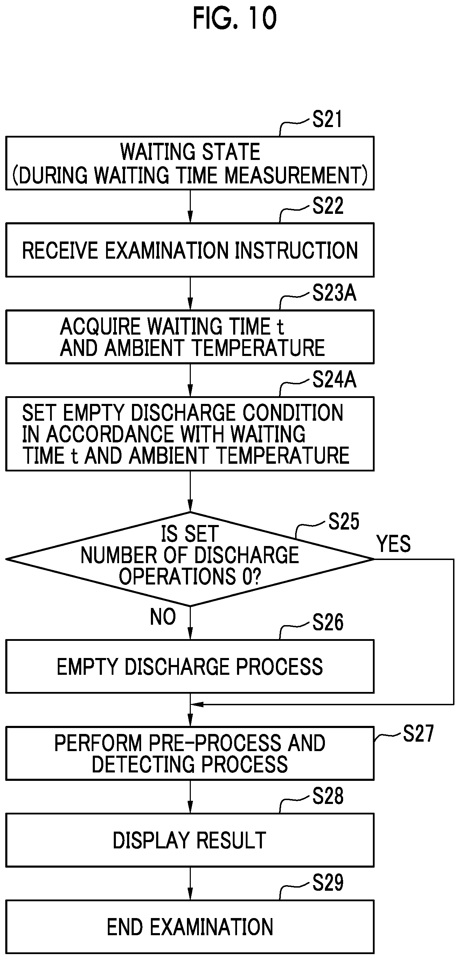

Fig. 10 is a diagram showing a second examination flow. The second examination flow is a process procedure in a case where the examination apparatus 10 is activated and the examination instruction is received in a waiting state (idle state) after the previous examination ends, similarly to the first examination flow. It should be noted that, in a case where the examination apparatus 10 is activated, the first heater 21 is set to 35°C and the second heater 22 is set to 38°C (first temperature T1) as an example. In the present example, the temperatures of the first heater 21 and the second heater 22 are not changed and are kept constant. It should be noted that, in Fig. 10, the same steps as those in the first examination flow shown in Fig. 6 are represented by the same reference numerals.

In the waiting state, the processor 16 measures the waiting time from the end of the previous examination (Step S21). In a case where the processor 16 receives the examination instruction for the next examination (Step S22), the processor 16 acquires the waiting time t from the end of the previous examination and the ambient temperature from the temperature sensor 29 (Step S23A).

The processor 16 sets the empty discharge condition including at least one of the number of discharge operations of the empty discharge of the empty discharge or the single discharge amount of the empty discharge in the empty discharge process in accordance with the waiting time t and the temperature sensor 29 (Step S24A). In the setting of the empty discharge condition (Step S24A), the processor 16 determines whether or not the combination of the waiting time t and the ambient temperature satisfies a preset condition. In a case where the combination of the waiting time t and the ambient temperature satisfies the preset condition, the processor 16 sets the number of discharge operations to 0. On the other hand, in a case where the combination of the waiting time t and the ambient temperature does not satisfy the preset condition, in the setting of the empty discharge condition (Step S24A), the processor 16 performs setting in which at least one of the number of discharge operations or the discharge amount is increased as the waiting time t is longer and at least one of the number of discharge operations or the discharge amount is increased as the ambient temperature decreases.

The preset condition for setting the number of discharge operations to 0 is, for example, a condition in which the waiting time t is shorter than a preset threshold value and the ambient temperature is within a preset range. Specific conditions are appropriately set, such as a case where the waiting time t is 10 minutes or less and the ambient temperature is 28°C or higher, and a case where the waiting time t is 20 minutes or less and the ambient temperature is 29°C or higher.

The waiting time t and the ambient temperature, and the map 50 (refer to Fig. 11) of the number of discharge operations and/or the discharge amount may be stored in the memory 17, and the processor 16 may determine the empty discharge condition with reference to the map 50. The map 50 shown in Fig. 11 is an example showing the number of discharge operations which are set by the waiting time t and the ambient temperature. In a case of referring to the map 50, for example, in a case where the waiting time is 40 minutes and the ambient temperature is 30°C, the number of discharge operations is set to 1.

Thereafter, in a case where the set number of discharge operations is 0 (Step S25: YES), the processor 16 performs the pre-process and the photodetecting process without performing the empty discharge process (Step S27). In a case where the set number of discharge operations is not 0 (Step S25: NO), the processor 16 controls the dispensing unit 14 such that the dispensing unit 14 performs the empty discharge process of empty-discharging the cleaning liquid 40 from the nozzle 14A in accordance with the set empty discharge condition (Step S26).

Then, the processor 16 controls the dispensing unit 14 such that the dispensing unit 14 performs the pre-process (Step ST11 to ST17 in Fig. 2), and controls the photodetector 15 such that the photodetector 15 performs the detecting process (Step ST18 in Fig. 2) (Step S27). It should be noted that, in the procedure of the chemiluminescent enzyme immunoassay described with reference to Fig. 2, the processes up to the second reaction (Step ST15 in Fig. 2) may be performed in parallel in a period from the reception of the examination instruction (Step S22) to the empty discharge process (Step S26). In particular, it is preferable that the empty discharge process (Step S26) is performed immediately before the second cleaning process (Step ST16 in Fig. 2), and the second cleaning process is performed immediately after the empty discharge process (Step S26).

The processor 16 detects a concentration of the examination target substance A on the basis of the received light signal from the photodetector 15, displays the examination result on the touch panel display 18 (Step S28), and ends the examination (Step S29). It should be noted that, after the end of this examination (Step S29), in a case where the examination of the next specimen is not performed, the examination apparatus 10 returns to the first waiting state (Step S21), and the processor 16 measures the waiting time.

In such a manner, by combining the waiting time t and the ambient temperature and executing the temperature control in accordance with both the waiting time t and the ambient temperature, it is possible to discharge the liquid, which has an appropriate temperature stabilized faster than in a case where the temperature control is executed in accordance with only the waiting time t, into the reaction cell R0.

Further, in the above-mentioned first examination flow and second examination flow, the processor 16 executes the empty discharge process as the temperature control corresponding to the waiting time t, but may execute, as the temperature control, a temperature setting change process of setting the heater (here, the second heater 22) to a second temperature T2 higher than the first temperature T1.

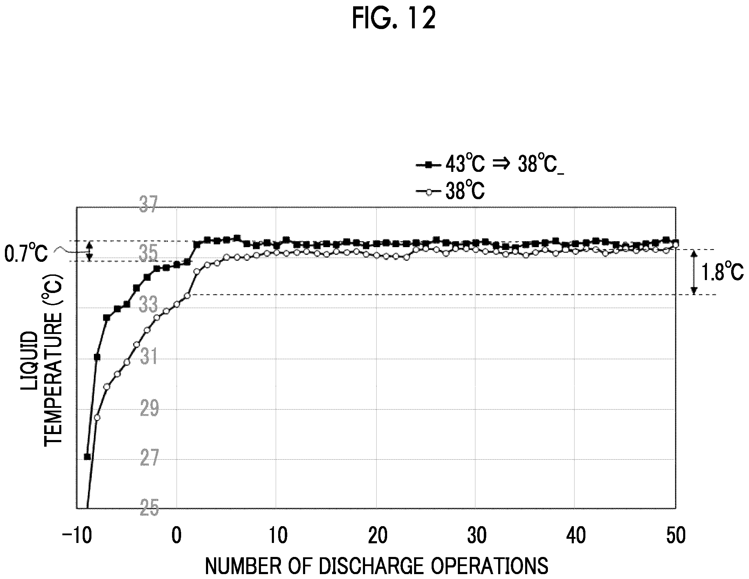

Fig. 12 shows a relationship between the number of discharge operations and the liquid temperature immediately after the waiting state in a case where the temperature of the second heater 22 is fixed at 38°C which is the first temperature T1 (broken line) and in a case where the temperature of the second heater 22 is once increased to 43°C which is the second temperature T2 and then the temperature setting change process of returning the temperature to 38°C which is the first temperature T1 is executed (solid line). The empty discharge conditions are common, and the empty discharge is performed 10 times (from the negative side of the horizontal axis to 0). In Fig. 12, "1" on the horizontal axis of the number of discharge operations corresponds to the discharge into the reaction cell R0 in the examination of the primary specimen (first specimen) after the waiting state.

As shown in Fig. 12, in a case where the second heater 22 is fixed to 38°C, the difference in liquid temperature between the first specimen and the fiftieth specimen in which the liquid temperature is sufficiently stable is 1.8°C. On the other hand, in a case where the temperature setting change process is executed, the liquid temperature difference between the first specimen and the fiftieth specimen is suppressed to 0.7°C. Further, in a case where the temperature setting change process is executed, the liquid temperature of the second specimen is stably maintained at a temperature close to the liquid temperature, but in a case where the second heater 22 is fixed to 38°C, the liquid temperatures in a range to the vicinity of the tenth specimen are not stably maintained.

As described above, in the examination apparatus 10, the processor 16 may execute the temperature setting change process of setting the temperature of the second heater 22 to the second temperature T2 higher than the first temperature T1, instead of the temperature control for executing the empty discharge process of empty-discharging the cleaning liquid 40, of which the temperature is decreased, from the dispensing unit 14 to a place other than the reaction cell R0 from the dispensing unit 14. Even in such a case, the processor 16 sets the second temperature T2 in accordance with the waiting time t. Therefore, the temperature of the discharged cleaning liquid 40 can be promptly stabilized, and the liquid (here, the cleaning liquid 40) having an appropriate temperature can be discharged into the reaction cell R0. Since it is possible to discharge the liquid at the appropriate temperature for each specimen into the reaction cell R0, highly reliable examination results can be obtained.

The second temperature T2 is preferably set to a higher temperature as the waiting time t is longer.

As shown in Fig. 3, after the examination ends, the liquid temperature of the cleaning liquid 40 in the pipe portion 20A gradually decreases as the waiting time t is longer. The lower the temperatures of the nozzle 14A and the pipe portion 20A at the start of the next examination, the greater the temperatures need to be increased to the target temperatures. Here, the following case will be considered: a case where the empty discharge condition is set to be constant and the discharging is started at the time point ta at which the waiting time is relatively long and the time point tb at which the waiting time is relatively short as shown in Fig. 3, that is, a case where the next examination is started. At the time point tb, the decrease in temperature of the cleaning liquid 40, the pipe portion 20A, and the nozzle 14A is larger than that at the time point ta. Therefore, the temperature needs to be increased by a larger amount in order to set the liquid temperature at the time of discharging of the cleaning liquid 40 to the target temperature. That is, in a case where the temperature setting of the second heater 22 is changed, by setting the second temperature T2 to be higher as the waiting time t is longer, it is possible to discharge the cleaning liquid 40, which has a temperature stabilized at an early stage, into the reaction cell R0 even in a case where the waiting time is long.

In the examination apparatus 10, as a third examination flow in a case where the examination is performed on the specimen 30, an examination flow in a case where the processor 16 executes the temperature control including the temperature setting change process in accordance with the waiting time t will be described. In the third examination flow, the empty discharge is performed under the preset empty discharge condition, and the empty discharge condition is not set in accordance with the waiting time t.

### (Third Examination Flow)

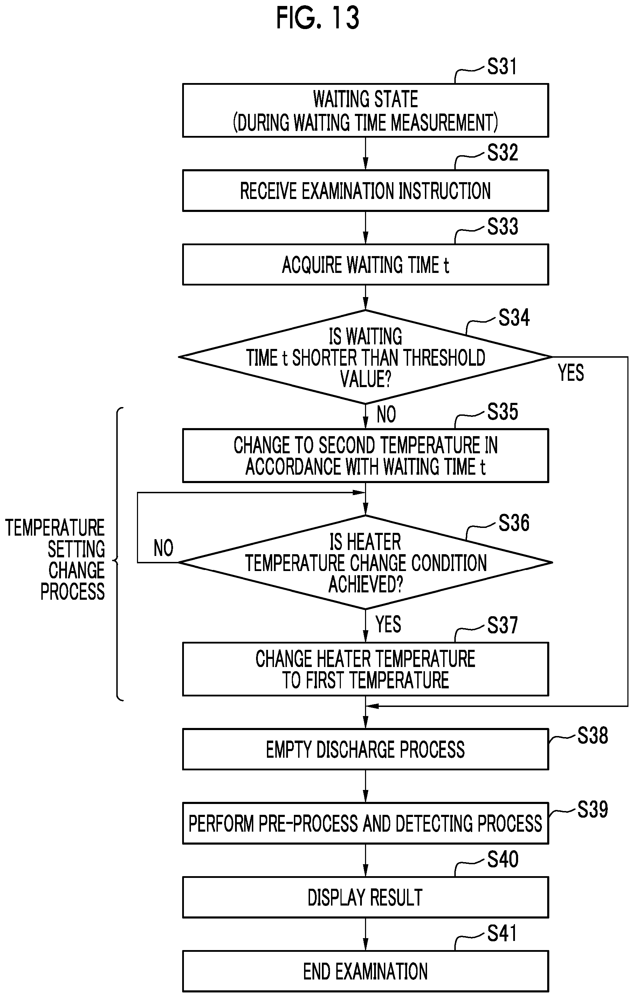

Fig. 13 is a diagram showing a third examination flow. The third examination flow is a process procedure in a case where the examination apparatus 10 is activated and the examination instruction is received in a waiting state (idle state) after the previous examination ends, similarly to the first examination flow and the second examination flow. It should be noted that, in a case where the examination apparatus 10 is activated, the first heater 21 is set to 35°C and the second heater 22 is set to 38°C (first temperature T1) as an example.

In the waiting state, the processor 16 measures the waiting time from the end of the previous examination (Step S31). In a case where the examination instruction for the next examination is received (Step S32), the processor 16 acquires the waiting time t from the end of the previous examination (Step S33).

The processor 16 determines whether or not the waiting time t is shorter than a preset threshold value β (Step S34). Here, in a case where the waiting time t is shorter than the threshold value β (Step S34: YES), the processor 16 controls the dispensing unit 14 such that the dispensing unit 14 performs the empty discharge process (Step S38). On the other hand, in a case where the waiting time t is equal to or longer than the threshold value β (Step S34: NO), the temperature setting change process is executed in accordance with the waiting time t (steps S35 to S37).

In the temperature setting change process, first, the processor 16 changes the temperature of the second heater 22 to the second temperature T2 higher than the first temperature T1 in accordance with the waiting time t (Step S35). The second temperature T2 is a temperature determined in accordance with the waiting time t, and is set to be higher as the waiting time t is longer and to be lower as the waiting time t is shorter. Thereafter, in a case where the heater temperature change condition is satisfied (Step S36: YES), the processor 16 changes the temperature of the second heater 22 to the original first temperature T1 (Step S37). It should be noted that, in the present example, the temperature of the first heater 21 is fixed.

Here, the heater temperature change condition is a condition appropriately determined, such as a condition after a preset predetermined time passed, a condition immediately before the second cleaning process, or a condition immediately before the start of the pre-process, which is a condition after the temperature of the second heater 22 is changed to the second temperature T2.

The processor 16 changes the temperature of the second heater 22 to the first temperature T1 and then controls the dispensing unit 14 such that the dispensing unit 14 performs the empty discharge process (Step S38).

After the empty discharge process (Step S38), the processor 16 controls the dispensing unit 14 such that the dispensing unit 14 performs the pre-process and controls the photodetector 15 such that the photodetector 15 performs the detecting process (Step S39). It should be noted that, in the procedure of the chemiluminescent enzyme immunoassay described with reference to Fig. 2, the processes up to the second reaction (Step ST15 in Fig. 2) may be performed in parallel in a period from the reception of the examination instruction (Step S32) to the empty discharge process (Step S38). In particular, it is preferable that the empty discharge process (Step S38) is performed immediately before the second cleaning process (Step ST16 in Fig. 2), and the second cleaning process is performed immediately after the empty discharge process (Step S38).

The processor 16 detects a concentration of the examination target substance A on the basis of the received light signal from the photodetector 15, displays the examination result on the touch panel display 18 (Step S40), and ends the examination (Step S41). It should be noted that, after the end of this examination (Step S41), in a case where the examination of the next specimen is not performed, the examination apparatus 10 returns to the first waiting state (Step S31), and the processor 16 measures the waiting time.

As a modification example of the third examination flow, the processor 16 may execute the temperature control in accordance with both the waiting time t and the ambient temperature by combining the waiting time t and the ambient temperature, similarly to the second examination flow.

As a fourth examination flow in a case of performing the examination of the specimen 30 in the examination apparatus 10, an examination flow in a case where the processor 16 executes the temperature control including the temperature setting change process in accordance with the waiting time t and the ambient temperature will be described. In the fourth examination flow, the empty discharge is performed under the preset empty discharge condition, and the empty discharge condition is not set in accordance with the waiting time t.

### (Fourth Examination Flow)

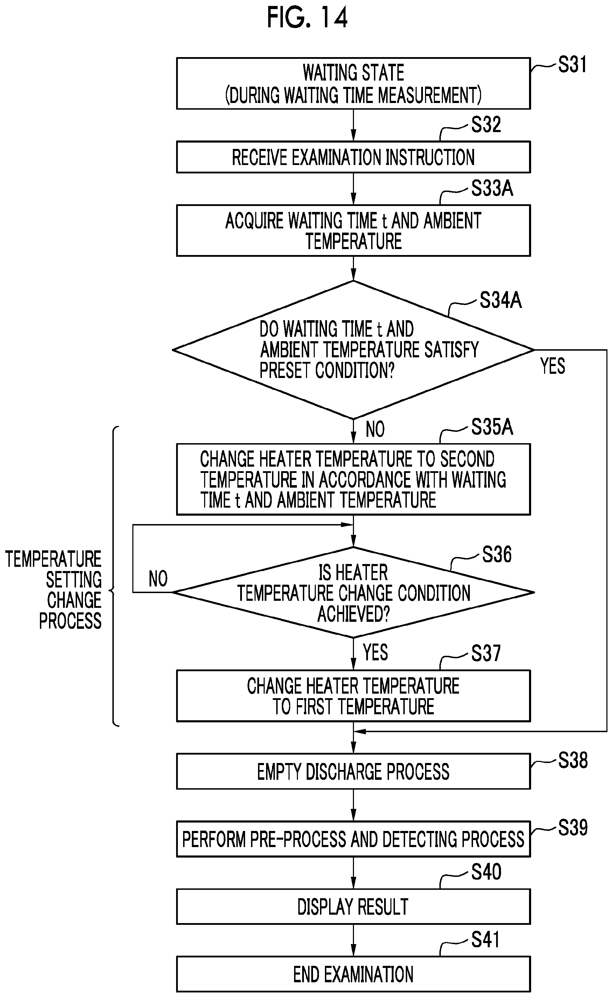

Fig. 14 is a diagram showing a fourth examination flow. The fourth examination flow is a process procedure in a case where the examination apparatus 10 is activated and the examination instruction is received in the waiting state (idle state) after the previous examination ends, similarly to the first examination flow, the second examination flow, and the third examination flow. It should be noted that, in a case where the examination apparatus 10 is activated, the first heater 21 is set to 35°C and the second heater 22 is set to 38°C (first temperature T1) as an example. It should be noted that, in Fig. 14, the same steps as those in the first examination flow shown in Fig. 13 are represented by the same reference numerals.

In the waiting state, the processor 16 measures the waiting time from the end of the previous examination (Step S31). In a case where the processor 16 receives the examination instruction for the next examination (Step S32), the processor 16 acquires the waiting time t from the end of the previous examination and the ambient temperature from the temperature sensor 29 (Step S33A).

The processor 16 determines whether or not the combination of the waiting time t and the ambient temperature satisfies the preset condition (Step S34A). Here, in a case where the combination of the waiting time t and the ambient temperature satisfies the preset condition (Step S34: YES), the processor 16 controls the dispensing unit 14 such that the dispensing unit 14 performs the empty discharge process (Step S38). On the other hand, in a case where the combination of the waiting time t and the ambient temperature does not satisfy the preset condition (Step S34A: NO), the temperature setting change process is executed in accordance with the waiting time t and the temperature sensor 29 (steps S35A to S37).

The preset condition used in Step S34A is, for example, a condition in which the waiting time t is shorter than a preset threshold value and the ambient temperature is within a preset range. Specific conditions are appropriately set, such as a case where the waiting time t is 10 minutes or less and the ambient temperature is 25°C or higher, and a case where the waiting time t is 20 minutes or less and the ambient temperature is 26°C or higher.

In the temperature setting change process, first, the processor 16 changes the temperature of the second heater 22 to the second temperature T2 higher than the first temperature T1 in accordance with the waiting time t and the ambient temperature (Step S35A). The second temperature T2 is a temperature determined in accordance with the waiting time t and the ambient temperature, and is set to be higher as the waiting time t is longer and to be lower as the waiting time t is shorter, and is set to be higher as the ambient temperature is lower and to be lower as the ambient temperature is higher. Thereafter, in a case where the heater temperature change condition is satisfied (Step S36: YES), the processor 16 changes the temperature of the second heater 22 to the original first temperature T1 (Step S37). It should be noted that, in the present example, the temperature of the first heater 21 is fixed.

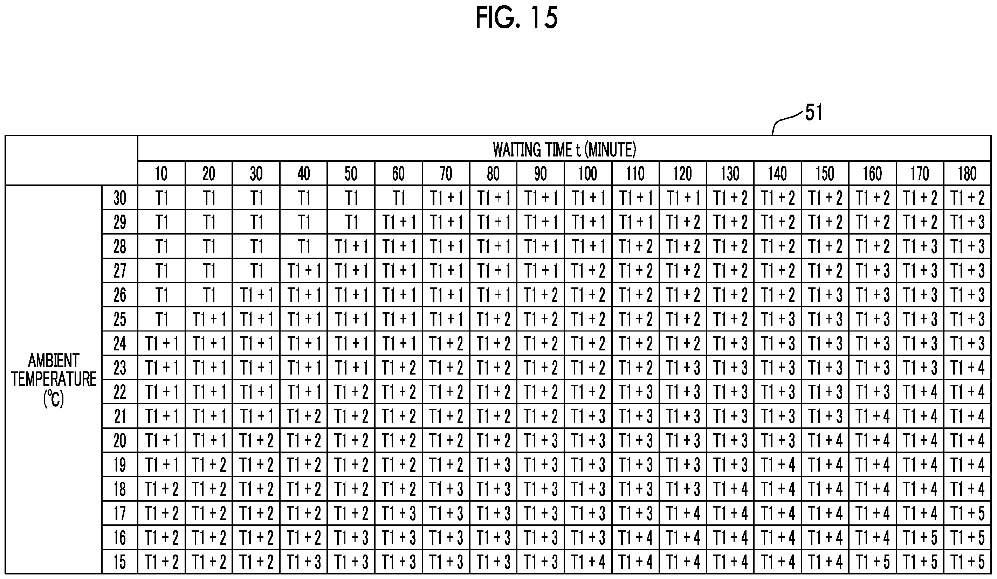

A map 51 (refer to Fig. 15) of the waiting time t and the ambient temperature and the second temperature T2 may be stored in the memory 17, and the processor 16 may set the second temperature T2 with reference to the map 51. The map 51 shown in Fig. 15 is an example showing the second temperature T2 which is set by the waiting time t and the ambient temperature. In a case of referring to the map 51, for example, in a case where the waiting time is 40 minutes and the ambient temperature is 25°C, the second temperature T2 is set to the first temperature T1+1°C. In the present example, since the first temperature T1 is 38°C, the second temperature T2 is 39°C.

Further, the heater temperature change condition is a condition appropriately determined, such as a condition after a preset predetermined time passed, a condition immediately before the second cleaning process, or a condition immediately before the start of the pre-process, which is a condition after the temperature of the second heater 22 is changed to the second temperature T2.

The processor 16 changes the temperature of the second heater 22 to the first temperature T1 and then controls the dispensing unit 14 such that the dispensing unit 14 performs the empty discharge process (Step S38).

After the empty discharge process (Step S38), the processor 16 controls the dispensing unit 14 such that the dispensing unit 14 performs the pre-process and controls the photodetector 15 such that the photodetector 15 performs the detecting process (Step S39). It should be noted that, in the procedure of the chemiluminescent enzyme immunoassay described with reference to Fig. 2, the processes up to the second reaction (Step ST15 in Fig. 2) may be performed in parallel in a period from the reception of the examination instruction (Step S32) to the empty discharge process (Step S38). In particular, it is preferable that the empty discharge process (Step S38) is performed immediately before the second cleaning process (Step ST16 in Fig. 2), and the second cleaning process is performed immediately after the empty discharge process (Step S38).

The processor 16 detects a concentration of the examination target substance A on the basis of the received light signal from the photodetector 15, displays the examination result on the touch panel display 18 (Step S40), and ends the examination (Step S41). It should be noted that, after the end of this examination (Step S41), in a case where the examination of the next specimen is not performed, the examination apparatus 10 returns to the first waiting state (Step S31), and the processor 16 measures the waiting time.

In such a manner, by combining the waiting time t and the ambient temperature and executing the temperature control in accordance with both the waiting time t and the ambient temperature, it is possible to discharge the liquid, which has an appropriate temperature stabilized faster than in a case where the temperature control is executed in accordance with only the waiting time t, into the reaction cell R0.

In the above description, the following has been described as an example. In the examination of the next specimen 30, the processor 16 executes, in accordance with the waiting time t or the waiting time t and the ambient temperature, the temperature control including any one of the empty discharge process or the temperature setting change process as the temperature control for compensating for the decrease in temperature of the liquid occurring in accordance with the waiting time t. As a further modification example, the processor 16 may execute, in accordance with the waiting time t or the waiting time t and the ambient temperature, the temperature control including both the empty discharge process and the temperature setting change process as the temperature control for compensating for the decrease in temperature of the first liquid that decreases in accordance with the waiting time t.

In a case where both the empty discharge process and the temperature setting change process of the heater are executed as the temperature control, it is possible to discharge a liquid having an appropriate temperature that is more promptly stabilized into the reaction container. Further, by increasing the temperature of the heater, the temperature of the cleaning liquid 40 can be increased promptly. Therefore, the number of empty discharge operations or the single discharge amount can be reduced. In such a case, the amount of the cleaning liquid 40 used can be reduced.

In a case where the examination apparatus 10 comprises the temperature sensor 29 that measures the ambient temperature, the processor 16 may execute the temperature setting change process in the waiting state of the examination apparatus 10.

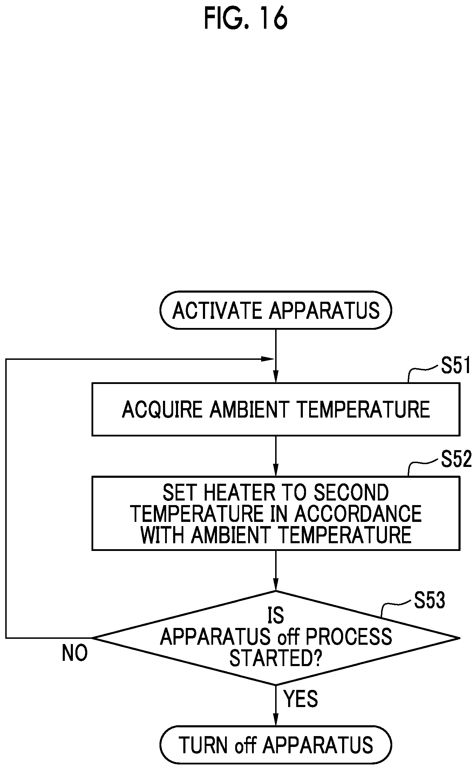

Fig. 16 shows a temperature setting change process procedure in the waiting state (idle state).

In a case where the power supply of the examination apparatus 10 is turned on and the examination apparatus 10 is activated, the processor 16 acquires the ambient temperature from the temperature sensor 29 (Step S51). Then, the temperature of the second heater 22 is set to the second temperature T2 in accordance with the ambient temperature (Step S52). It should be noted that, at the time of the activation, the first heater 21 is set to a predetermined temperature (for example, 35°C).

Then, until an OFF process of the examination apparatus is started (Step S53: NO), the acquisition (Step S51) of the ambient temperature and the step (Step S52) of setting the temperature of the second heater 22 to the new second temperature T2 in accordance with the ambient temperature are repeated. Then, in a case where the OFF process of the examination apparatus is started (Step S53: YES), the power supply of the examination apparatus 10 is turned off.

This process is executed in the waiting state and is not executed at the time of the examination of the specimen 30. At the time of the examination, for example, the first examination flow, the second examination flow, the third examination flow, the fourth examination flow, or the like described above is applied.

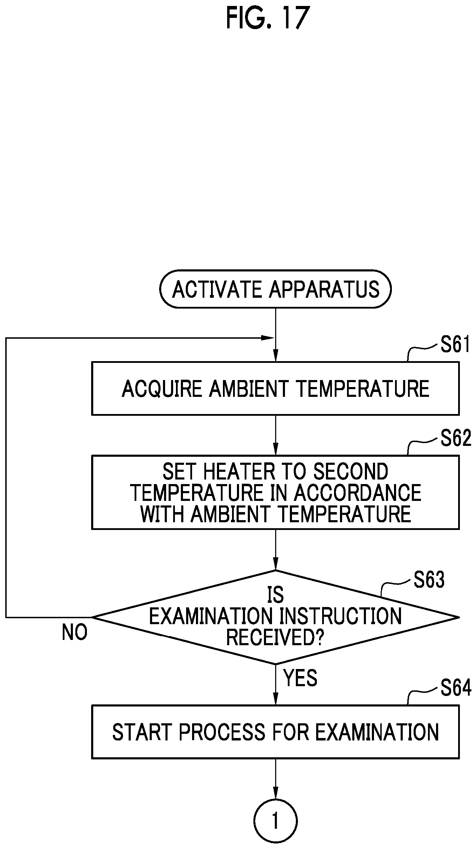

Further, an example of the flow relating to the temperature control of the cleaning liquid 40 for the second cleaning process from immediately after the activation of the examination apparatus 10 to the OFF of the power supply will be described with reference to Figs. 17 to 19.

First, Fig. 17 shows a flow from the activation of the operation of the examination apparatus 10 to the reception of the first examination. In a case where the power supply of the examination apparatus 10 is turned on and the examination apparatus 10 is activated, the processor 16 acquires the ambient temperature from the temperature sensor 29 (Step S61). Then, the temperature of the second heater 22 is set to the second temperature T2 in accordance with the ambient temperature (Step S62). It should be noted that, at the time of the activation, the first heater 21 is set to a predetermined temperature (for example, 35°C).

Then, until the examination instruction is received (Step S63: NO), the acquisition (Step S61) of the ambient temperature and the step (Step S62) of setting the temperature of the second heater 22 to the new second temperature T2 in accordance with the ambient temperature are repeated. Then, in a case where the examination instruction is received (Step S63: YES), the process for the examination is started (Step S64).

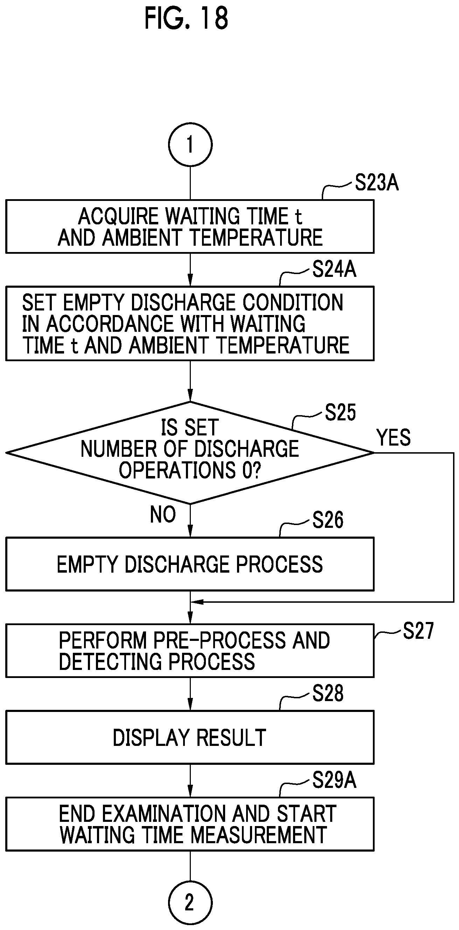

Fig. 18 shows an examination flow after the examination instruction is received. The present examination flow corresponds to Step S23A to Step S28 of the second examination flow. Following Step S62 shown in Fig. 17, the processor 16 acquires the waiting time t and the ambient temperature (Step S23A). The subsequent process is the same as the second examination flow until the step (Step S28) of displaying the examination result.

It should be noted that, as the examination flow after the examination instruction is received, Step S23 to S28 of the first examination flow, Step S33 to S40 of the third examination flow, Step S33A to S40 of the fourth examination flow, or the like may be applied.

After the examination result is displayed, the examination ends, and the processor 16 starts to measure the waiting time t at the same time as the examination ends (Step S29A). Then, the examination apparatus 10 proceeds to the waiting state.

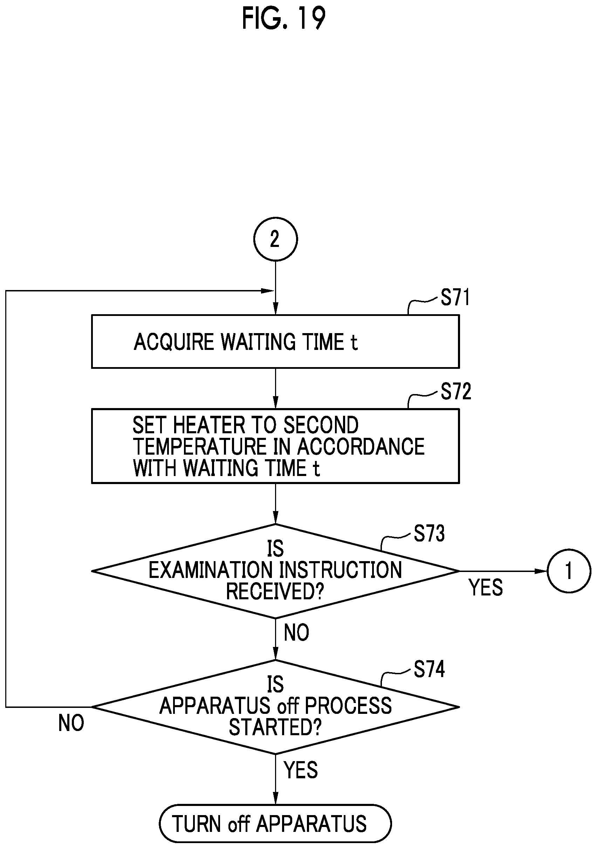

Fig. 19 shows a flow of temperature control of the cleaning liquid 40 in the waiting state.

In the waiting state, the processor 16 acquires the waiting time t (Step S71) and sets the temperature of the second heater 22 to the second temperature T2 in accordance with the waiting time t (Step S72). Thereafter, in a case where the examination instruction is not received (Step S73: NO) and the apparatus OFF process is not started (Step S74: NO), the acquisition (Step S71) of the waiting time t and the setting (Step S72) of the new second temperature T2 are repeated.

The second temperature T2, which is set in accordance with the waiting time t, is set to, for example, 39°C in a case where the waiting time is 5 minutes, 39.5°C in a case where the waiting time is 10 minutes, 40°C in a case where the waiting time is 30 minutes, and the like. That is, the second temperature T2 is set to be higher as the waiting time t is longer.

In a case where the examination instruction is received (Step S73: YES), the process proceeds to the examination flow shown in Fig. 18. Further, even in a case where there is no examination instruction (Step S73: NO), in a case where the apparatus OFF process is started (Step S74: YES), the power supply of the examination apparatus 10 is turned off.

The above description has been given as an example of the flow of the temperature control of the cleaning liquid 40 for the second cleaning process from immediately after the activation of the examination apparatus 10 to the time at which the power supply is turned off.

In the examination apparatus 10 according to the above-mentioned embodiment, the configuration has been described in which the temperature control is performed on the cleaning liquid (an example of the first liquid) supplied from the cleaning liquid tank (an example of the liquid tank) to the nozzle 14A via the pipe in a case where the cleaning liquid is discharged into the reaction cell R0 through the nozzle. That is, a case where the liquid discharged from the nozzle 14A to the reaction cell R0 and the liquid supplied from the liquid tank to the nozzle 14A are the same has been described. On the other hand, the temperature control of the present disclosure can be applied even in a case where the liquid to be discharged from the nozzle is different from the liquid supplied from the liquid tank to the nozzle via the pipe.

For example, the reaction cell R0 used in the above-mentioned examination apparatus 10 is provided in a cartridge RC together with a plurality of cells R1 to R4 for accommodating the reagents 36 to 39 and the like (refer to Fig. 20). The cartridge RC shown in Fig. 20 is a single-use type cartridge that is used once for one specimen 30. In the cartridge RC as an example, the cell R1 accommodates a buffer solution 36. The cell R2 accommodates the labeling reagent 37 including the label S in which the second binding substance B2 to specifically bind to the target substance is modified. The cell R3 accommodates the first luminescent reagent 38, and the cell R4 accommodates the second luminescent reagent 39.

As described above, the dispensing unit 14 performs a process of dispensing and mixing of liquids such as the above-mentioned buffer solution 36, the labeling reagent 37, the first luminescent reagent 38, the second luminescent reagent 39, and the cleaning liquid 40. Therefore, the dispensing unit 14 comprises, in addition to the nozzle 14A for dispensing the cleaning liquid 40, a nozzle for suctioning and dispensing each of the reagents 37 to 39 and the like. Fig. 20 shows, as one of the nozzles, a nozzle 14B for dispensing the labeling reagent 37. The nozzle 14B can be moved between the cleaning pod 26 and between the reaction cell R0 and the cell R2.

Further, the examination apparatus 10 comprises a heater 42 that heats each reagent. The heater 42 controls the temperature of each of the reagents 36 to 39. In a case where the target temperature at the time of discharging into the reaction cell R0 is, for example, 37°C, the temperature of each of the reagents 36 to 39 is controlled to, for example, 38°C in consideration of a decrease in temperature at the time of suctioning and dispensing. Each of the reagents 36 to 39 is suctioned from each of the cells R1 to R4 by a dedicated nozzle and dispensed into the reaction cell R0. Hereinafter, for example, the suctioning and dispensing of the labeling reagent 37 using the nozzle 14B will be described.

In a case where the specimen 30 is continuously examined, the nozzle 14B repeats the suction and the discharge of the labeling reagent 37. Therefore, the temperature of the nozzle 14B is substantially equal to the reagent temperature. On the other hand, in a case where there is a waiting time t from the examination of one specimen 30 to the examination of the next specimen 30, the temperature of the nozzle 14B decreases in accordance with the waiting time t. The relationship between the waiting time t and the temperature of the nozzle 14B is the same as the relationship between the waiting time t shown in Fig. 3 and the liquid temperature of the cleaning liquid 40 held in the nozzle 14A. That is, as the waiting time t is longer, the temperature of the nozzle 14B is closer to the temperature inside the apparatus.

In a case where the labeling reagent 37 of which the temperature is controlled at 38°C is suctioned using the nozzle 14B of which the temperature is decreased after the waiting time t, the heat of the temperature-controlled labeling reagent 37 is taken away by the nozzle 14B. Thus, the temperature of the labeling reagent 37 is decreased. Therefore, the temperature at the time of dispensing into the reaction cell R0 may be significantly deviated from the target temperature of 37°C, which may affect the examination result.

The processor 16 may execute the temperature control executed in the second cleaning process described above in a case of dispensing the cleaning liquid 40 in a case of dispensing the labeling reagent 37. That is, the temperature control of warming the nozzle 14B may be executed before the labeling reagent 37 is suctioned through the nozzle 14B.

The nozzle 14B suctions the labeling reagent 37 from the cell R2 of the cartridge RC and discharges the labeling reagent 37 into the reaction cell R0. On the other hand, the nozzle 14B is connected to the pipe 20b connected to the cleaning liquid tank 12 on the base end side, and the cleaning liquid 40 can be supplied to the nozzle 14B from the base end side. Before the labeling reagent 37 is suctioned through the nozzle 14B, the processor 16 supplies the cleaning liquid 40 heated by the first heater 21 and the second heater 22 to the nozzle 14B and performs empty discharge thereof, thereby warming the nozzle 14B. The processor 16 warms the nozzle 14B and then performs the suction and the discharge of the labeling reagent 37 through the nozzle 14B. The nozzle 14B is a nozzle through which the labeling reagent 37 is discharged as the first liquid, and is a nozzle through which the cleaning liquid 40 is empty-discharged as the second liquid. It should be noted that, by suctioning air before suctioning the labeling reagent 37 through the nozzle 14B to form a gap between the air and the cleaning liquid 40, the labeling reagent 37 can be discharged into the reaction cell R0 without being mixed with the cleaning liquid 40.

In the empty discharge process, the processor 16 sets the empty discharge condition under which the cleaning liquid 40 is empty-discharged in accordance with the waiting time t, or sets the temperature of the second heater 22 to the second temperature higher than the first temperature in accordance with the waiting time in the temperature setting change process of the heater. The setting of the empty discharge condition and the temperature setting of the heater may be performed in the same manner as the temperature control performed at the time of dispensing the cleaning liquid 40 in the second cleaning process described above. As described above, the processor 16 may perform the temperature control of compensating for the decrease in temperature of the nozzle 14B occurring in accordance with the waiting time t. Thereby, the decrease in temperature of the labeling reagent 37 in a case of being suctioned through the nozzle 14B can be suppressed, and the labeling reagent 37, in which the decrease in temperature from the target temperature is suppressed, can be supplied to the reaction cell R0.

As described above, the examination apparatus 10 may comprise the dispensing unit 14 including the nozzle 14B that discharges the first liquid (in the above-mentioned example, the labeling reagent 37) into the reaction cell R0 and the liquid tank (in the above-mentioned example, the cleaning liquid tank 12) that stores the second reagent (in the above-mentioned example, the cleaning liquid 40) different from the first liquid. In such a case, the second liquid is used in order to compensate for the decrease in temperature of the nozzle for discharging the first liquid occurring in accordance with the waiting time t. Also in such a case, by applying the temperature control of the present disclosure, the first liquid having a temperature with less dissociation from the target temperature can be discharged into the reaction cell. Therefore, the reliability of the examination can be improved.

In the above description, the dispensing of the labeling reagent 37 has been described, but the same temperature control may be performed in a case of dispensing the buffer solution 36, the first luminescent reagent 38, and the second luminescent reagent 39.

In the above-mentioned embodiment, the immunological analysis apparatus that detects the examination target substance included in the specimen by using the antigen-antibody reaction was described as an example of the examination apparatus 10. As a matter of course, the technology of the present disclosure can be used for an examination apparatus other than the immunological analysis apparatus. Further, the chemiluminescent immunoassay method has been described as an example of the method of detecting the examination target substance, but the present disclosure is not limited to this method, and may be applied to other methods.

Further, in the above-mentioned embodiment, as a hardware structure of the processor, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific process, such as an application specific integrated circuit (ASIC), in addition to a CPU that is a general-purpose processor configured to execute software (program) to function as various processing units.

Further, the above-mentioned process may be executed by one of various processors or may be executed by a combination of two or more processors (for example, a combination of a plurality of FPGAs or a CPU and an FPGA) of the same type or different types. The plurality of processing units may be configured of one processor. As an example where a plurality of processing units are composed of one processor, there is a form in which a processor that realizes all functions of a system including a plurality of processing units into one integrated circuit (IC) chip is used, such as system-on-chip (SOC).

Further, the hardware structure of the processors is, more specifically, an electric circuit (circuitry) in which circuit elements such as semiconductor elements are combined.

The disclosure of JP2022-060858 filed on March 31, 2022 is incorporated herein by reference in its entirety. All documents, patent applications, and technical standards described in the present specification are incorporated into the present specification by reference to the same extent as in a case where the individual documents, patent applications, and technical standards were specifically and individually stated to be incorporated by reference.

## Claims

1. An examination apparatus that sequentially performs examination on a plurality of specimens, the examination apparatus comprising:
a dispensing unit that includes a nozzle through which a first liquid is discharged into a reaction cell in which a specimen is accommodated;
a liquid tank that stores the first liquid or a second liquid different from the first liquid;
a heater that is disposed closer to a side of the liquid tank than the dispensing unit and heats the first liquid or the second liquid; and
a processor that controls the dispensing unit and the heater,
wherein the processor is configured to
maintain a temperature of the heater at or above a predetermined first temperature during a period from end of the examination of one specimen to start of the examination of a next specimen, and measure a waiting time from the end of the examination of the one specimen to the examination of the next specimen, and
execute, in accordance with the waiting time, a temperature control which is for compensating for a decrease in temperature of the first liquid or a decrease in temperature of the nozzle caused by the waiting time, the temperature control including at least one of an empty discharge process of empty-discharging the first liquid or the second liquid from the dispensing unit to a place other than the reaction cell or a temperature setting change process of setting the heater to a second temperature higher than the first temperature.

2. The examination apparatus according to claim 1,
wherein the processor is configured to set an empty discharge condition, which includes at least one of the number of discharge operations of the empty discharge or a single discharge amount of the empty discharge in the empty discharge process, in accordance with the waiting time.

3. The examination apparatus according to claim 2,
wherein the processor is configured to, in the setting of the empty discharge condition, increase at least one of the number of discharge operations or the discharge amount as the waiting time increases.

4. The examination apparatus according to claim 2,
wherein the processor is configured to set the number of discharge operations to 0 in a case where the waiting time is shorter than a preset threshold value in the setting of the empty discharge condition.

5. The examination apparatus according to any one of claims 1 to 4,
wherein the processor is configured to set the second temperature to be higher as the waiting time increases in the temperature setting change process.

6. The examination apparatus according to claim 1 or 2, further comprising:
a temperature sensor that measures an ambient temperature,
wherein the processor is configured to execute the temperature control in accordance with the waiting time and the ambient temperature.

7. The examination apparatus according to claim 6 which cites claim 2,
wherein the processor is configured to increase at least one of the number of discharge operations or the discharge amount as the waiting time increases and to increase at least one of the number of discharge operations or the discharge amount as the ambient temperature decreases in the setting of the empty discharge condition.

8. The examination apparatus according to claim 6 which cites claim 2,
wherein the processor is configured to set the number of discharge operations to 0 in a case of satisfying a condition in which the waiting time and the ambient temperature are preset in the setting of the empty discharge condition.

9. The examination apparatus according to any one of claims 6 to 8,
wherein the processor is configured to set the second temperature to be higher as the waiting time increases and to set the second temperature to be higher as the ambient temperature decreases in the temperature setting change process.

10. The examination apparatus according to any one of claims 1 to 9,
wherein the liquid tank is a liquid tank that stores the first liquid,
the heater is a heater that heats the first liquid, and
the processor is configured to execute, in accordance with the waiting time, a temperature control which is for compensating for a decrease in temperature of the first liquid caused by the waiting time, the temperature control including at least one of the empty discharge process of empty-discharging the first liquid from the dispensing unit to the place other than the reaction cell or a setting change process of increasing a set temperature of the heater.

11. The examination apparatus according to claim 10,
wherein the first liquid is a cleaning liquid used for a cleaning process of separating an unreacted component from a reacted component in a reaction liquid obtained by reacting the specimen with a reagent.
